# EUROPÄISCHE PATENTANMELDUNG

(11) **EP 3 715 843 A1**
(43) Veröffentlichungstag der Anmeldung: **30.09.2020**
(21) Anmeldenummer: 20165516.4
(22) Anmeldetag: 25.03.2020
(51) Int. Cl.: G01N 33/00, H03M 1/18

(54) **MESSVORRICHTUNG**

(30) Priorität: 26.03.2019 DE 102019204183
(71) Anmelder: FRAUNHOFER-GESELLSCHAFT zur Förderung der angewandten Forschung e.V., 80686 München (DE)
(72) Erfinder: PETRAUTZKI, Dirk, 91058 Erlangen (DE); WASTIAN, Raphael, 91058 Erlangen (DE); WANK, Florian, 96465 Neustadt/Coburg (DE); HÜLß, Markus, 91058 Erlangen (DE)
(74) Vertreter: Pfitzner, Hannes

(57) **Zusammenfassung**

Eine Messvorrichtung umfasst einen elektro-chemischen Sensor, eine Messelektronik sowie einen Controller. Der Sensor gibt in Abhängigkeit einer in der Umgebung des Sensors vorliegenden Gaskonzentration ein Messsignal aus. Die Messelektronik umfasst eine dynamisch skalierbare Verstärkerstufe sowie einen A/D-Wandler. Die dynamisch skalierbare Verstärkerstufe verstärkt entsprechend eines Skalierungsfaktors das Messsignal, der A/D-Wandler digitalisiert das verstärkte Messsignal. Der Controller ist ausgebildet, den Skalierungsfaktor für die Verstärkerstufe einzustellen. Beansprucht wird zudem ein Verfahren zur Steuerung einer entsprechenden Messvorrichtung, sowie ein Computerprogramm zur Durchführung des Verfahrens auf einem Computer.

## Beschreibung

Ausführungsbeispiele der vorliegenden Erfindung beziehen sich auf eine Messvorrichtung sowie ein Verfahren zur Steuerung einer Messvorrichtung. Bevorzugte Ausführungsbeispiele beziehen sich auf eine Messvorrichtung mit einer Messelektronik, die ein analoges Frontend mit dynamischer Skalierbarkeit aufweist.

Bisher kommen für die Gasmessung - am Beispiel von Stickstoffen - Geräte wie z. B. der Siemens Ultramat 23 zum Einsatz. Eine derartige Vorrichtung ist nicht nur sehr groß, sondern auch wartungsintensiv und insgesamt sehr teuer. Weitere Informationen zu derartigen Geräten finden sich unter https://w3.siemens.com/mcms/sensor-systems/de/prozessanalytik/extraktive-kontinuierliche-prozesse-gasanalytik/seiten/ ultramat-23.aspx und https://www.umweltbundesamt.de/sites/default/files/medien/ 3593/dokumente/e stickstoffoxide.pdf. Es gibt auch zahlreiche Geräte, die bautechnisch bedingt eine gewisse Größe (meist Gehäuse für 19"-Racks) aufweisen und dem Standard DIN EN 14211 für Referenzverfahren für Stickstoffoxid mittels Chemilumineszenz entsprechen. Weitere Informationen finden sich unter http://www,environnement-sa.com/products-page/en/mir9000-multi-gas-analyzer-infra-red-gfc-cld-chemilumine scence/ oder unter https://w3.siemens. com/mcms/sensorsystems/de/ prozessanalytik/extraktive-kontinuierliche-prozesse-gas analytik/seiten/(ultramat-23.aspx. Die DIN ISO 13964 definiert eine Referenzmethode für die Ozonbestimmung mittels eines UV-photometrischen Verfahrens. All diese Verfahren sind zwar sehr genau, aber wie bereits oben angesprochen nachteilhaft in Bezug auf Bauraum, Wartungskosten und Anschaffungskosten.

Bei diesen Verfahren bzw. allgemein bei der Bestimmung von Gaskonzentrationen in der Luft werden elektronische Signalaufbereitungen verwendet. Hierzu existieren beispielsweise folgende Standardlösungen: http://www.alphasense.com/WEB1213/wpcontent/uploads/2013/07/AAN 105-03.pdf sowie https://www_{.}analog.com/media/en/ reference-design-documentation/reference-designs/CN0234.pdf.

Ausgehend von dem Stand der Technik und den Nachteilen besteht die Aufgabe der vorliegenden Erfindung darin, eine Vorrichtung zur Bestimmung einer Gaskonzentration zu schaffen, die einen verbesserten Kompromiss aus Messgenauigkeit, Auswertungsaufwand und Kosteneffizienz schafft.

Die Aufgabe wird durch den Gegenstand der unabhängigen Patentansprüche gelöst.

Ausführungsbeispiele der vorliegenden Erfindung schaffen eine Messvorrichtung mit einem elektro-chemischen Sensor, einer Messelektronik sowie einem Controller. Der elektro-chemische Sensor ist ausgebildet, um in Abhängigkeit einer in der Umgebung des Sensors vorliegenden Gaskonzentration ein Messsignal auszugeben. Die Messelektronik umfasst eine optionale Vorverstärkerstufe, eine dynamisch skalierbare Verstärkerstufe sowie einen A/D-Wandler. Die optionale Vorverstärkerstufe führt eine Vorverstärkung des Messsignals durch. Die dynamisch skalierbare Verstärkerstufe verstärkt entsprechend eines Skalierungsfaktors das vorverstärkte Messsignal. Der A/D-Wandler digitalisiert das verstärkte Messsignal. Der (Mikro-)Controller ist ausgebildet, den Skalierungsfaktor für die Verstärkungsstufe entsprechend einzustellen.

Entsprechend Ausführungsbeispielen empfängt der Controller (der beispielsweise Teil der digitalen Stufe sein kann) das digitalisierte Messsignal und bestimmt in Abhängigkeit von einer Auswertung des digitalisierten Messsignals den Skalierungsfaktor.

Ausführungsbeispielen der vorliegenden Erfindung liegt die Erkenntnis zugrunde, dass durch die Verwendung eines skalierbaren (analogen) Verstärkers in der Messelektronik Messsignale in einem sehr weiten Messbereich (von Picoampere bis in den zweistelligen Mikroamperbereich) verarbeitet werden können. Vergleichbar große Messsignale, die zu einem großen Messbereich führen, entstehen beispielsweise aufgrund der chemischen Reaktivität bei einem Temperaturanstieg. Insgesamt entsteht so der Vorteil eines deutlich reduzierten Preises gegenüber alternativen Technologien zur Gaskonstellationsmessung. Auch ist eine gute Anbindung in weitere Systeme möglich. Diese Elektronik mit analogem Frontend und digitalem Backend ist vorteilhafterweise in sehr geringen Umfang störanfällig. Entsprechend dem bevorzugten Ausführungsbeispiel kann die Sensorik (wie auch die Elektronik) als Austauschmodul eingesetzt werden. Der Austausch, z.B. der Sensoren, ist aufgrund des Elektrolytverbrauchs im Sensor sinnvoll, um eine hohe Messgenauigkeit zu erhalten. Durch die Austauschbarkeit wird der Wartungsaufwand reduziert. Entsprechend Ausführungsbeispielen können mehrere elektro-chemische Sensoren und mehrere zugehörige Messelektroniken vorgesehen sein. Der Controller ist hierbei dann entsprechend einem Ausführungsbeispiel ausgebildet, für die mehreren dynamisch skalierbaren Verstärkerstufen der mehreren Messelektroniken unterschiedliche Skalierungsstufen parallel oder seriell auszuwählen.

Entsprechend einem Ausführungsbeispiel kann der Controller ein Speicher mit Kalibrierwerten umfassen, der ausgebildet ist, um die digitalisierten Messwerte entsprechend den gespeicherten Kalibrierwerten zu verarbeiten. Die Kalibrierwerte können sich von Messsensor zu Messsensor unterscheiden, wobei es denkbar ist, dass die Kalibrierwerte mit jedem Messsensor (jedem modular austauschbaren Messsensor) mitgeliefert werden und so auch herstellungsbedingte Unterschiede in den Kalibrierwerten Berücksichtigung finden.

Entsprechend einem Ausführungsbeispiel kann die Messelektronik eine rückseitige Schirmung und/oder eine Platine mit einer rückseitigen Schirmung umfassen. Diese rückseitige Schirmung dient einerseits zur Vermeidung von elektronischen Signalen, kann aber auch zusätzlich Kühlmittel (z. B. einen Kühlkörper) aufweisen, der ausgebildet ist, die Temperatur der Messelektronik zu reduzieren.

Ein zusätzliches Ausführungsbeispiel umfasst ein Verfahren zur Steuerung einer Messvorrichtung mit dem elektro-chemischen Sensor sowie der Messelektronik. Das Verfahren umfasst den Schritt des Festlegens des Skalierungsfaktors und/oder des Festlegens des Skalierungsfaktors in Abhängigkeit einer Analyse des digitalisierten Messsignals. Das Verfahren kann auch Computer-implementiert sein.

Weiterbildungen sind in den Unteransprüchen definiert. Ausführungsbeispiele der vorliegenden Erfindung werden Bezug nehmend auf die beiliegenden Zeichnungen erläutert. Es zeigen:
- Fig. 1: eine Messvorrichtung mit einem elektro-chemischen Sensor sowie einer Messelektronik gemäß einem Basisausführungsbeispiel; und
- Fig. 2: eine Messelektronik vergleichbar zu der Messelektronik aus Fig. 1 mit optionalen Merkmalen gemäß einem erweiterten Ausführungsbeispiel.

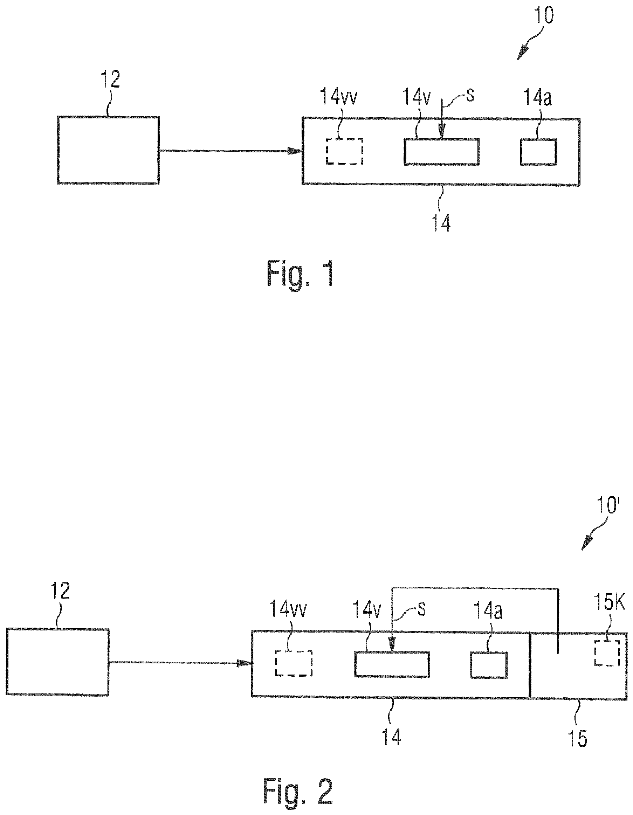

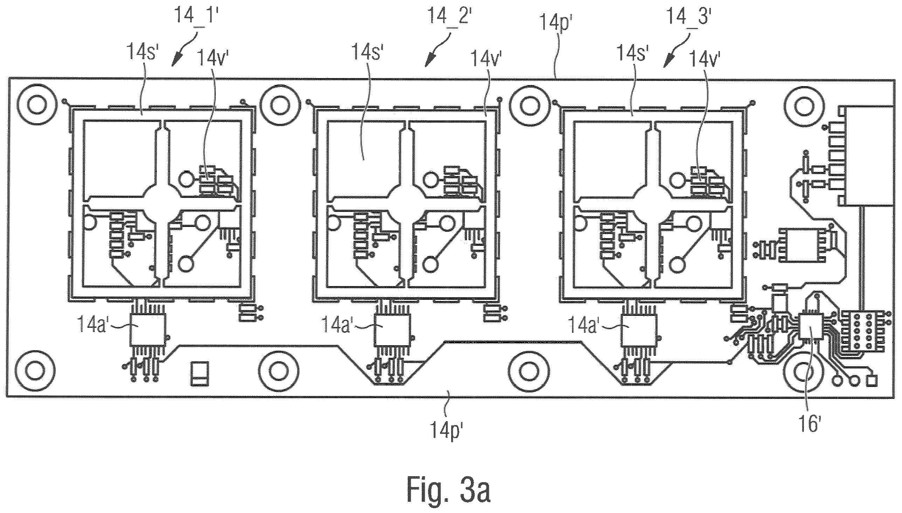

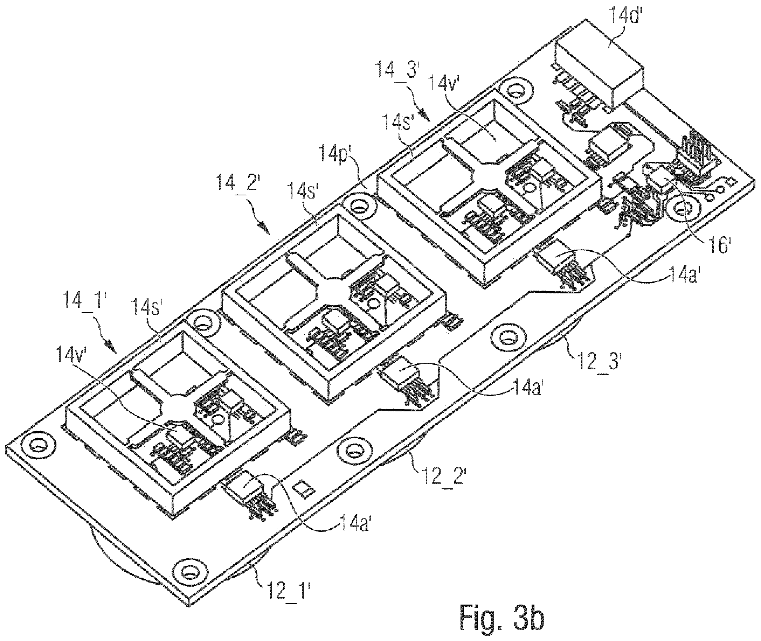

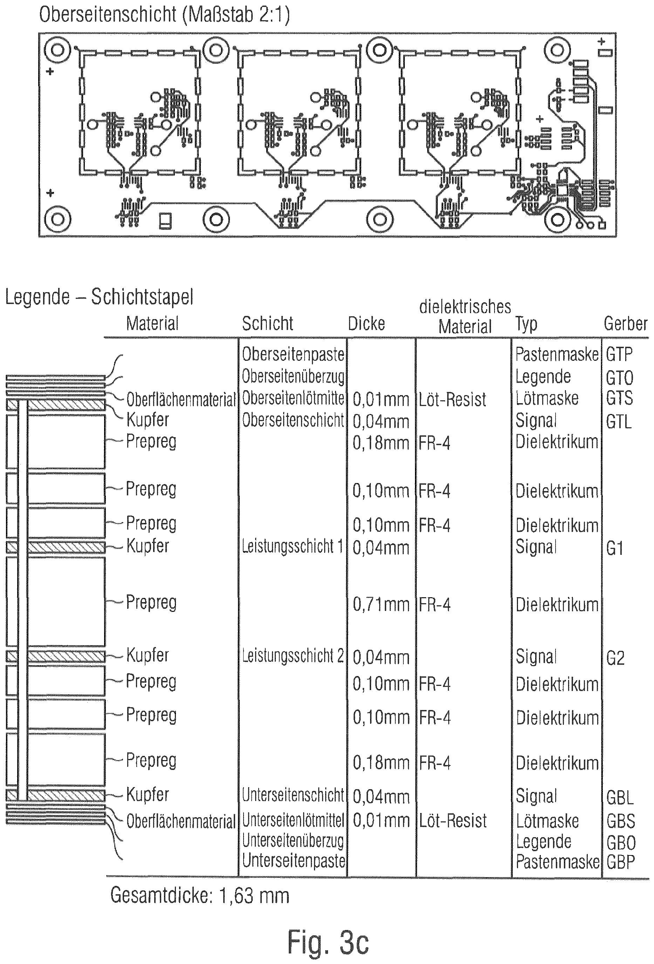

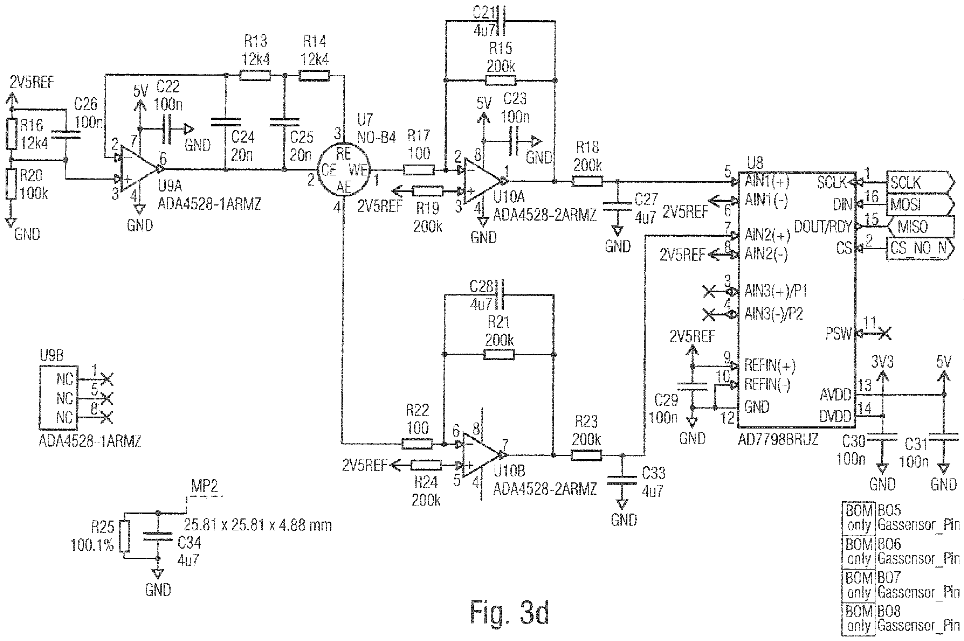

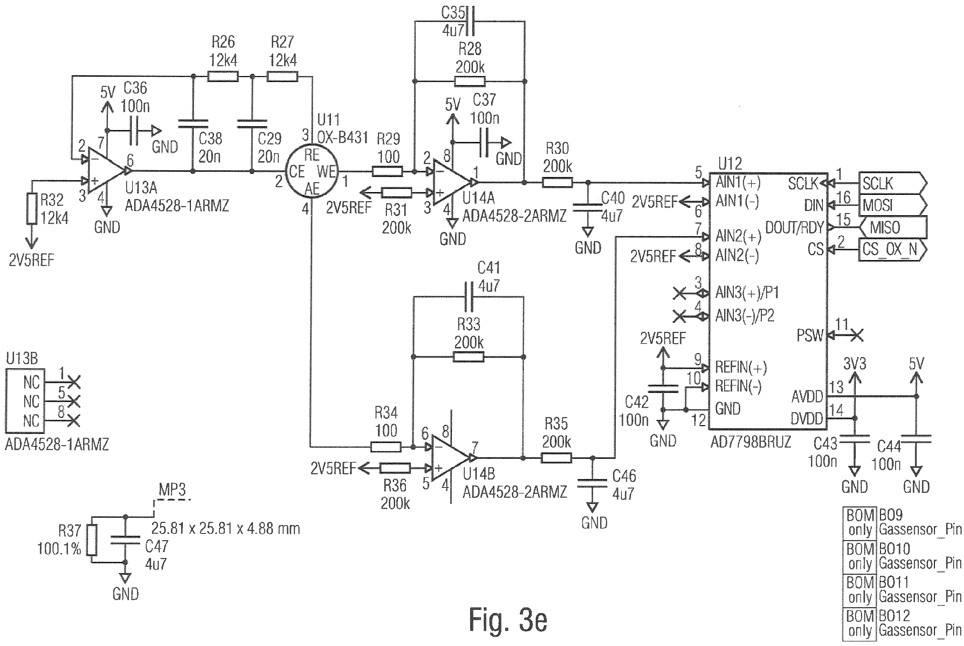

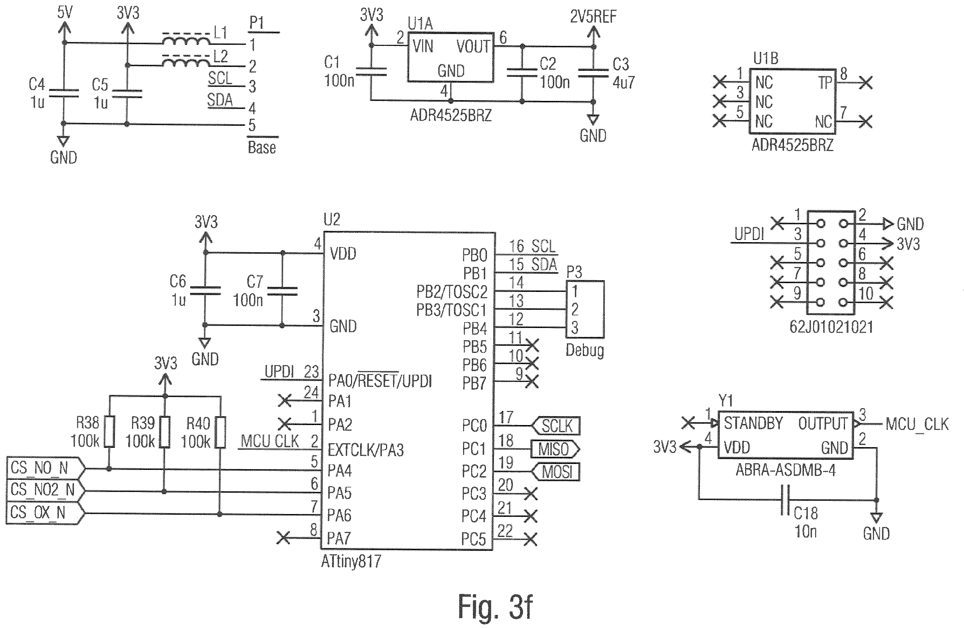

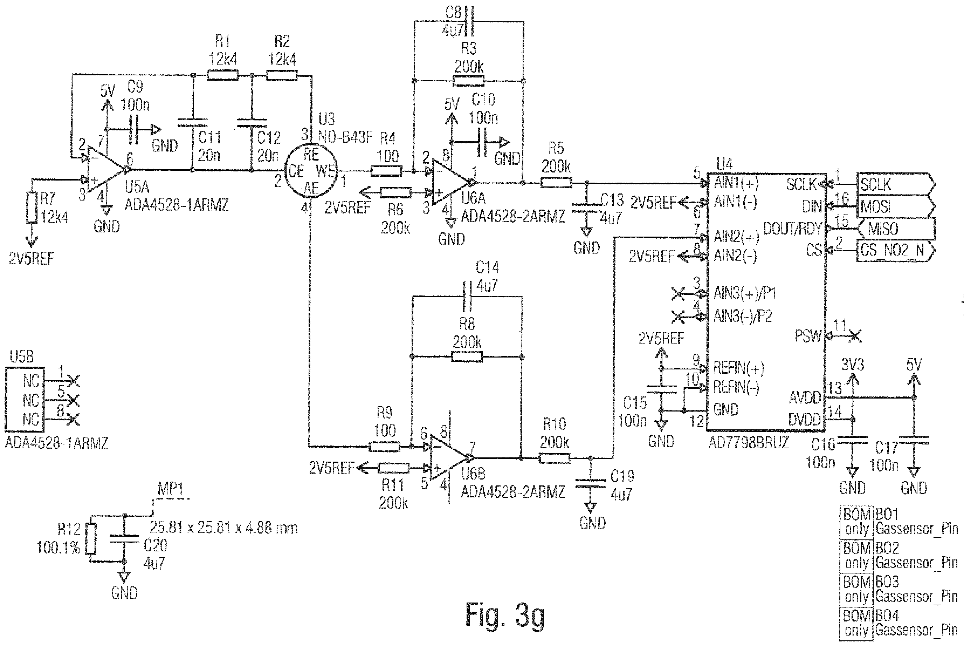

Fig. 1 zeigt eine Messvorrichtung 10 mit einem elektro-chemischen Sensor 12 und einer Messelektronik 14. Die Messelektronik 14 umfasst in der hier einfachsten Implementierung eine Verstärkerstufe 14v und eine Digitalisierungsstufe 14a. Optionaler Weise kann eine der Verstärkerstufe 14v vorgelagerte Vorverstärkerstufe 14vv vorgesehen sein.

Bei dem Sensor 12 kann es sich beispielsweise um einen kommerziell erhältlichen elektro-chemischen Sensor handeln, der ausgebildet ist, in der Umgebungsluft die aktuelle Konstellation von Ozon, Kohlenstoffdioxid oder Kohlenstoffmonoxid zu bestimmen. Dieser gibt seine Messwerte, z. B. analoge Signale mit Strömen im Bereich von Nanoamper an die Messelektronik 14 aus. In dieser Messelektronik wird unter Zuhilfenahme der Elemente 14vv und 14v bzw. nur 14v das Messsignal verstärkt bzw. vorverstärkt. Die Verstärkung erfolgt entsprechend einem Skalierungsfaktor s, der den Verstärker 14v in Bezug auf seine Verstärkung steuert. Das durch den Verstärker 14v verstärkte Messsignal wird dann in dem A/D-Wandler 14a digitalisiert.

In diesem Ausführungsbeispiel erfolgt die Steuerung des Verstärkers 14v anhand von Skalierungsfaktoren, die z. B. durch eine externe Steuerung (nicht dargestellt) gesteuert wird. Durch das dynamische Vorgeben des Faktors s ist es möglich, dass ein sehr großer Messbereich bis in den zweistelligen Mikroamperbereich abgedeckt wird. Dies ist deshalb vorteilhaft, da die elektro-chemische Reaktivität bei einem Temperaturanstieg stark zunimmt.

Das Festlegen des Faktors S kann z.B. wie folgt erfolgen:
- Durchführen einer (Test-)Messung (erste Messung) mit einen niedrigen Skalierungsfaktor S (z.B. 10%, 25%, 30% oder 40% des Aussteuergrads des AD-Wandlers 14a; und
- Festlegen des Skalierungsfaktor S für die weiter Messung so, dass der AD-Wandler 14a mindestens zu 90% oder zu 80% oder zu 50% ausgesteuert wird.

Dieser Ansatz hat den Vorteil, dass im ersten Schritt ein Übersteuern das AD-Wandlers 14a verhindert wird und schnell ein zweiter Messwert mit guter Auflösung aufgrund ausreichender Austeuerung des AD-Wandlers 14a erhalten werden kann.

Fig. 2 zeigt eine Messvorrichtung 10' mit der elektro-chemischen Zelle 12 sowie einer Messelektronik 14'. Diese umfasst wiederum die Einheiten 14v und 14a sowie die optionale Vorverstärkerstufe 14vv und ist dabei um einen Controller 15 erweitert. Dieser Controller 15 analysiert das durch den A/D-Wandler 14a digitalisierte Messsignal und variiert in Abhängigkeit hiervon den Verstärkungsfaktor s. Beispielsweise wird der Faktor s erhöht, wenn durch den Controller 15 festgestellt wird, dass eine weitere Verstärkung notwendig ist oder reduziert, wenn der A/D-Wandler 14a übersteuert wird.

Entsprechend weiteren Ausführungsbeispielen kann der Controller 15 Kalibriermittel 15k umfassen, die einen Speicher aufweisen können, in welchem Kalibrierdaten gespeichert sind. Ausgehend von diesen Kalibrierdaten können beispielsweise durch die Kalibriermittel 15k die digitalisierten Signale weiterprozessiert werden. Alternativ bzw. additiv wäre es auch denkbar, dass ausgehend von den Kalibrierdaten die Kalibriermittel 15k die Verstärkung s entsprechend anpassen. Anders ausgedrückt heißt es, dass der Skalierungsfaktor s von den Kalibrierdaten abhängen kann. Die Kalibrierdaten können für unterschiedliche Sensortypen des Sensors 12 gespeichert sein, aber auch herstellungsbedingte Unterschiede der gleichen Sensoren berücksichtigen. Wenn man davon ausgeht, dass der Sensor 12 als modular austauschbarer Sensor implementiert ist, können so Kalibrierdaten, die mit dem Sensor mitgeliefert sind bzw. die ausgehend von einer eindeutigen Zuordnung des Sensors eingelesen werden, Verwendung finden und ermöglichen so den Betrieb der Sensorvorrichtung 10' ohne vorherige erneute Kalibrierung, auch wenn gerade ein Modulaustausch erfolgt ist. Entsprechend Ausführungsbeispielen erfolgt die Kommunikation mit dem Sensormodul 12 digital, so dass eine einfache Systemintegration möglich ist.

Entsprechend einem Ausführungsbeispiel kann der analoge Teil der Steuerelektronik 14, d. h. also die Elemente 14vv, 14v und auch das Element 14a rückseitig geschirmt sein. Diese Schirmung ist mit der Platine verbunden und reduziert den Einfluss von elektromagnetischen Störfeldern. Gleichzeitig ermöglicht eine derartige Schirmung oder auch eine um ein Kühlmittel erweiterte Schirmung die Gewährleistung von gleichmäßiger und stabiler Temperaturverteilung zwischen dem Schaltungsteil für verschiedene Sensorelektroden, was zu stabilisierten Messwerten führt. Der Einsatz von präzisen Dünnschichtwiderständen hält das Temperaturrauschen besonders gering.

Entsprechend einem weiteren Ausführungsbeispiel kann die Messvorrichtung mehrere Sensoren 12 und mehrere parallele Messelektroniken 14 umfassen. In diesem Fall können auch mehrere Controller 15 vorgesehen sein, wobei prinzipiell ein Controller 15 reicht, der alle Skalierungsfaktoren s für die unterschiedlichen Verstärkerstufen 14v der unterschiedlichen Elektroniken 14 festlegt.

Die Messvorrichtung kann ein digitales Backend (digitale Auswertung) aufweisen, das ausgebildet ist, um das digitalisierte Messsignal oder mehrere digitalisierte Messsignale zu puffern und/oder um das digitalisierte Messsignal oder das digitalisierte, gepufferte Messsignal oder die digitalisierten, gepufferten Messsignale zu versenden und/oder um das digitalisierte Messsignal zu filtern, mitteln oder zu prozessieren. Das digitales Backend umfasst hierzu z.B. einen Prozessor und eine LAN- oder Funk-Schnittstelle.

An dieser Stelle sei angemerkt, dass die Messungen (z.B. erste Messung zur Ermittlung von S und weitere Messung oder Messungen einer Messreihe) beispielsweise 1 oder 1,5 ms beabstandet sind.

Sowohl auf dem Analog Frontend, als auch auf der im Gerät enthaltenen nachgelagerten Recheneinheit (und auch auf einem nachgelagerten Backend in Form einer Datenbank und Servern) können entsprechend Ausführungsbeispielen ein oder mehrere Algorithmen zur digitalen Filterung und/oder Vorverarbeitung der Messwerte ablaufen. Dies könnte z.B. ein einfaches arithmetisches Mittel, ein Medianfilter, ein gleitender Mittelwert, ein Sinc-Filter etc. sein.

Entsprechend Ausführungsbeispielen kann die Gassensorik dahingehend modularisiert werden, dass jeder Sensor auch ein eigenes AnalogFrontend mit sich bringt. Dies ermöglicht die Messung von verschiedenen nach Anwendungsfall kombinierten Gasen. D.h. im Vergleich zu vorher gibt es nicht mehr ein AnalogFrontend für die drei Gase NO, NO2 und O3 und man ist nicht mehr auf genau diese Kombination festgelegt. Diese modularen Sensoriken - bestehend aus Gassensor + analogen Frontend - sind wie bereits vorher wieder mit einer digitalen ID ausgestattet, so dass ein Austausch vom System selbst bemerkt werden kann und das System selbst darauf reagiert, um den Wartungsaufwand zu minimieren.

Bei einer Variante mit einer Controller ID kann die Baugruppe als solche eineindeutig im Gesamtaufbaukontext zugeordnet werden, sodass Kalibrierdaten und Alterung für das System stets zuzuordnen sind, woraufhin der Zustand im Lebenszyklus jederzeit ermittelbar ist.

Entsprechend Ausführungsbeispielen ist das analoge Frontend dahingehend entworfen, um selbst einen möglichst geringen Temperaturdrift zu haben. D.h. die elektronischen Bauteile wurden so ausgesucht, dass diese auf hohe/niedrige Temperaturen ein möglichst geringes Rauschen zum eigentlichen Nutzsignal hinzufügen. Ausführungsbeispiel hierzu werden nachfolgend erläutert:
Beispielsweise wurde auf dem analogen Frontend eine galvanische Trennung hinzugefügt, um die Signalqualität weiter zu verbessern.

Das Gehäuse des Gesamtsystems kann z.B. aus Aluminium bestehen, was durch seine Eigenschaften zu geringerer EMV-Einstrahlungen und damit verbundenen Störungen führt und somit wieder der Signalqualität zugutekommt.

Das System ist - entsprechend Ausführungsbeispielen, wenn z.B. eine entsprechende nachgelagerte Recheneinheit im Gerät verbaut ist - Over-the-Air-Update-fähig. D.h. sowohl die Software auf der nachgelagerten Recheneinheit, als auch die Software auf dem Mikrocontroller des AnalogFrontends kann nachträglich verändert werden. D.h. es könnte z.B. das Filterverhalten etc. verändert werden.

Entsprechend Ausführungsbeispielen kann auf der nachgelagerte Recheneinheit im Gerät (oder im Backend) eine digitale Kompensation von Luftdruck, Lufttemperatur und Luftfeuchtigkeit vorgenommen werden, um die Qualität der Messwerte weiter zu verbessern.

Entsprechend Ausführungsbeispielen kann an das Messsystem eine weitere Einheit mit separaten Bedienelementen und Display zur Steuerung des Messsystems (z.B. Start eines Kalibrationsmodus) und zur Darstellung der Messwerte angeschlossen sein.

Mittels dieser weiteren Einheit, oder auf andere Weise von außen getriggert (z.B. vom Backend aus) kann entsprechend Ausführungsbeispielen ein Algorithmus zur Kalibration des Gerätes angestoßen werden. Dies dient dazu, den Kalibrierprozess im Labor zu vereinfachen/verkürzen.

Entsprechend Ausführungsbeispielen kann das System anstatt mit einem Luftstrom, der durch einen Lüfter erzeugt wird, auch mit einem Luftstrom der durch eine Luftpumpe erzeugt wird betrieben werden. Die Luftpumpe liegt in der Reihenfolge hinter der Gasmessung, so dass diese nicht von eventuellen Verunreinigungen und/oder Wärmeentwicklung der Pumpe beeinträchtigt wird.

Entsprechend Ausführungsbeispielen kann in Kombination mit entsprechenden Luftschläuchen die Entnahme des Probengases somit nun örtlich entfernt vom Gerät erfolgen.

Entsprechend Ausführungsbeispielen kann die Temperierung des Probengases dahingehend optimiert sein, dass das Gas vorher durch ein Labyrinth-System geführt wird, welches wiederrum direkt mit einem von Luft umströmten Kühler und/oder von einem Peltierelement temperiert wird.

Unabhängig davon, ob zur Temperierung nur der bisherige Gaskanal oder das Labyrinth-System zum Einsatz kommt, kommen für beide Varianten verschiedenen Materialien in Betracht, welche wiederum Einfluss auf die Messqualität haben können. So können der Gaskanal/das Labyrinth-System aus Kunststoff oder aus elektropoliertem Edelstahl sein.

Für ein Luftansaugrohr-/schlauch kommt entsprechend Ausführungsbeispielen ebenfalls der Einsatz von verschiedenen Materialien (Kunststoff oder elektropolierter Edelstahl) in Betracht, welche wiederum Einfluss auf die Messqualität haben können.

Entsprechend Ausführungsbeispielen kann der Gaskanal / das Labyrinth-System mittels einer Dämmung wärmeisoliert sein. Die Temperierung des restlichen Systems (exkl. Gaskanal/Labyrinth) kann über Außenluft mit Lüfter und/oder zusätzlichem Peltierelement oder ohne zusätzliche Maßnahmen realisiert sein.

Entsprechend Ausführungsbeispielen ist das System, im Gegensatz zu herkömmlichen Systemen, bewusst nicht nur für dein Einsatz bei einem Temperaturbereich von 0 - 30°C ausgelegt, sondern kann auch darüber hinaus betrieben werden.

Entsprechend Ausführungsbeispielen kann sowohl auf dem Analog Frontend, als auch auf der im Gerät enthaltenen nachgelagerten Recheneinheit (und auch auf einem nachgelagerten Backend in Form einer Datenbank und Servern) ein Algorithmus zur Kompensation der Alterung der Sensoren laufen, um den Zeitraum in dem die Messwerte eine ausgezeichnete Qualität haben zu verlängern.

Entsprechend Ausführungsbeispielen kann es ein Verfahren (z.B. im Backend) zum Einsatz kommen, welches anhand der Kalibrationswerte zweier durchgeführter Kalibrationen die Qualität der zwischen diesen zwei Zeitpunkten aufgenommenen Messwerte nachträglich verbessert, da durch diese beiden Fixpunkte nun Alterung der Sensoren in diesem Zeitraum genau bestimmt werden kann.

Ein weiteres Ausführungsbeispiel bezieht sich auf ein Verfahren zum Betreiben der Messvorrichtung 10 bzw. 10'. Dieses Verfahren umfasst im Wesentlichen den Schritt des Analysierens des Messsignals bzw. digitalisierten Messsignals (hier im Controller 15) und des Bestimmens des Skalierungsfaktors s.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahingehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Der Datenträger, das digitale Speichermedium oder das computerlesbare Medium sind typischerweise gegenständlich und/oder nicht-vergänglich bzw. nicht-vorübergehend.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahingehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahingehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die hierin beschriebenen Vorrichtungen können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Vorrichtungen, oder jedwede Komponenten der hierin beschriebenen Vorrichtungen können zumindest teilweise in Hardware und/oder in Software (Computerprogramm) implementiert sein.

Die hierin beschriebenen Verfahren können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Verfahren, oder jedwede Komponenten der hierin beschriebenen Verfahren können zumindest teilweise durch Hardware und/oder durch Software ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Messvorrichtung (10, 10') mit folgenden Merkmalen:
elektro-chemischer Sensor (12), der in Abhängigkeit einer in der Umgebung des Sensors (12) vorliegenden Gaskonzentration ein Messsignal ausgibt;
eine Messelektronik (14), die eine dynamisch skalierbare Verstärkerstufe (14v) sowie einen AD-Wandler umfasst, wobei die dynamisch skalierbare Verstärkerstufe (14v) entsprechend eines Skalierungsfaktors (S) das Messsignal verstärkt und der AD-Wandler das verstärkte Messsignal digitalisiert; und
einen Controller (15), der ausgebildet ist, den Skalierungsfaktor (S) für die Verstärkerstufe (14v) einzustellen; wobei der Controller (15) ausgebildet ist, um das digitalisierte Messsignal zu empfangen und in Abhängigkeit von einer Auswertung des digitalisierten Messsignals den Skalierungsfaktor (S) zu bestimmen.

2. Messvorrichtung (10, 10') gemäß Anspruch 1, wobei das Bestimmen das Durchführen einer ersten Messung zur Ermittlung des Skalierungsfaktors (S) mit einen niedrigen Skalierungsfaktor (S) umfasst; oder
wobei das Bestimmen das Durchführen einer ersten Messung zur Ermittlung des Skalierungsfaktors (S) mit einen niedrigen Skalierungsfaktor (S) umfasst, wobei der niedrige Skalierungsfaktor (S) für die erste Messung so gewählt ist, dass der AD-Wandler maximal zu 10% oder 25% oder 30% oder 40% ausgesteuert wird.

3. Messvorrichtung (10, 10') gemäß Anspruch 2, wobei der Skalierungsfaktor (S) für die weiter Messung so gewählt ist, dass der AD-Wandler mindestens zu 90% oder zu 80% oder zu 50% ausgesteuert wird.

4. Messvorrichtung (10, 10') gemäß einem der vorigen Ansprüche, wobei die Messvorrichtung (10, 10') mehrere elektro-chemische Sensoren (12) und mehrere zugehörige Messelektroniken (14) umfasst.

5. Messvorrichtung (10, 10') gemäß Anspruch 4, wobei der Controller (15) ausgebildet ist für die mehreren dynamisch skalierbaren Verstärkerstufen (14v) der mehreren Messelektroniken (14) unterschiedliche Skalierungsstufen auszuwählen.

6. Messvorrichtung (10, 10') gemäß einem der vorigen Ansprüche, wobei der Controller (15) einen Speicher mit Kalibrierwerten umfasst und ausgebildet ist, um die digitalisierten Messwerte entsprechend den gespeicherten Kalibrierwerten zu verarbeiten.

7. Messvorrichtung (10, 10') gemäß einem der vorigen Ansprüche, wobei die Messelektronik (14) eine Vorverstärkerstufe (14vv) umfasst, die das Messsignal für die Verstärkungsstufe vorverstärkt.

8. Messvorrichtung (10, 10') gemäß einem der vorigen Ansprüche, wobei die Messelektronik (14) eine rückseitige Schirmung und/oder eine Platine mit einer rückseitigen Schirmung aufweist.

9. Messvorrichtung (10, 10') gemäß Anspruch 8, wobei die rückseitige Schirmung Kühlmittel umfasst, die ausgebildet sind, um die Temperatur der Messelektronik (14) zu reduzieren.

10. Messvorrichtung (10, 10') gemäß einem der vorigen Ansprüche, wobei der elektro-chemische Sensor (12) und/oder elektro-chemische Sensoren (12) modular austauschbar sind.

11. Messvorrichtung (10, 10') gemäß einem der vorigen Ansprüche, wobei die Messelektronik (14) modular austauschbar sind.

12. Messvorrichtung (10, 10') gemäß einem der vorigen Ansprüche, wobei die Messvorrichtung (10, 10') ein digitales Backend aufweist, das ausgebildet ist, um das digitalisierte Messsignal oder mehrere digitalisierte Messsignale zu puffern und/oder um das digitalisierte Messsignal oder das digitalisierte, gepufferte Messsignal oder die digitalisierten, gepufferten Messsignale zu versenden und/oder um das digitalisierte Messsignal zu filtern, mitteln oder zu prozessieren.

13. Verfahren zur Steuerung einer Messvorrichtung (10, 10') mit einem elektro-chemischen Sensor (12), der in Abhängigkeit einer in der Umgebung des Sensors (12) vorliegenden Gaskonzentration Messsignale ausgibt, und eine Messelektronik (14) mit einer dynamisch skalierbaren Verstärkerstufe (14v) und einem AD-Wandler, wobei die dynamisch skalierbare Verstärkerstufe (14v) entsprechend eines Skalierungsfaktors (S) das Messsignal verstärkt und der AD-Wandler das verstärkte Messsignal digitalisiert;
mit folgenden Schritten:
Festlegen des Skalierungsfaktors (S) und/oder Festlegen des Skalierungsfaktors (S) in Abhängigkeit einer Analyse des digitalisierten Messsignals;
wobei in Abhängigkeit von einer Auswertung des digitalisierten Messsignals der Skalierungsfaktor (S) bestimmt wird.

14. Verfahren nach Anspruch 13, wobei das Festlegen das Durchführen einer ersten Messung mit niedrigem Skalierungsfaktor (S) umfasst und der Skalierungsfaktor (S) abhängig von der Analyse für die weitere Messung gesteigert wird; oder
wobei das Festlegen das Durchführen einer ersten Messung mit niedrigem Skalierungsfaktor (S) umfasst und der Skalierungsfaktor (S) abhängig von der Analyse gesteigert wird, wobei der niedrige Skalierungsfaktor (S) für die erste Messung so gewählt ist, dass der AD-Wandler maximal zu 10% oder 25% oder 30% oder 40% ausgesteuert wird.

15. Messvorrichtung (10, 10') gemäß Anspruch 14, wobei der Skalierungsfaktor (S) für die weiter Messung so gewählt ist, dass der AD-Wandler mindestens zu 90% oder zu 80% oder zu 50% ausgesteuert wird.

16. Computerprogramm zur Durchführung des Verfahrens nach Anspruch 13 oder 14, wenn das Programm auf einem Computer abläuft.
